# FASCICULE DE BREVET EUROPEEN

(11) **EP 3 718 534 B1**
(45) Date de publication et mention de la délivrance du brevet: **11.11.2026**
(21) Numéro de dépôt: 20163759.2
(22) Date de dépôt: 17.03.2020
(51) Int. Cl.: A61K 9/00, A61K 31/00, A61K 47/10, A61K 47/38, A61P 31/04

(54) **COMPOSITION PHARMACEUTIQUE CONTENANT DE L ÉTHANOL ET UN ANTIBIOTIQUE ET APTE À FORMER UNE MASSE SOLIDE AU CONTACT AVEC UNE SOLUTION SALINE - PRODUIT ET DISPOSITIF DE CONCENTRATION ET RELARGAGE DE PROXIMITÉ ASSOCIÉS**
PHARMAZEUTISCHE ZUSAMMENSETZUNG MIT ETHANOL UND EINEM ANTIBIOTIKUM, DAS BEIM KONTAKT MIT EINER KOCHSALZLÖSUNG EINE FESTE MASSE BILDET, ENTSPRECHENDES PRODUKT UND ENTSPRECHENDE VORRICHTUNG ZUR KONZENTRATION UND FREISETZUNG IN DER NÄHE
PHARMACEUTICAL COMPOSITION CONTAINING ETHANOL AND AN ANTIBIOTIC, AND SUITABLE FOR FORMING A SOLID MASS IN CONTACT WITH A SALINE SOLUTION - ASSOCIATED PRODUCT AND DEVICE FOR CONCENTRATION AND LOCAL DELIVERY

(30) Priorité: 01.04.2019 FR 1903446
(43) Date de publication de la demande: 07.10.2020
(73) Titulaire: Gelnesis, 14610 Cairon (FR)
(72) Inventeur: LE BAIL, Mcihel, 14610 CAIRON (FR)
(74) Mandataire: Santarelli

(56) Documents cités:
- WO-A1-2017/175081
- PHAECHAMUD THAWATCHAI ET AL: "Solvent exchange-inducedin situforming gel comprising ethyl cellulose-antimicrobial drugs", INTERNATIONAL JOURNAL OF PHARMACEUTICS, ELSEVIER, NL, vol. 494, no. 1, 21 August 2015 (2015-08-21), pages 381 - 392, XP029276816, ISSN: 0378-5173, DOI: 10.1016/J.IJPHARM.2015.08.047

## Description

La présente invention concerne une composition pharmaceutique contenant de l'éthanol et un antibiotique ; cette composition est apte à former une masse solide au contact d'une solution saline (composant majoritaire d'un tissu physiologique). La présente invention concerne également un produit de combinaison pouvant servir à la préparation de la composition de l'invention et son utilisation pour la formation d'un implant. La présente invention concerne également la composition pour son utilisation comme traitement de maladies répondant aux antibiotiques et ciblant notamment des sites infectieux, des kystes ou d'autres maladies entrainant des modifications pathologiques tissulaires.

Le document WO 03/087108 A1 décrit une composition pharmaceutique qui contient de l'éthylcellulose et de l'éthanol à 95%. Cette composition présente l'avantage de former un solide lorsqu'elle vient en contact avec une solution saline ; elle forme donc une masse solide lorsqu'elle est injectée dans un organisme.

Le document WO 2017/175081 A1 décrit la même composition que précitée mais suggère de la mélanger avec un antibiotique. Le but recherché est la diffusion de l'antibiotique au niveau du site infectieux dans lequel la composition a été injectée. Le document précité montre que l'éosine migre bien de la masse solide formée par contact avec une solution saline vers le milieu extérieur à la masse, soit la solution saline. Le document ne comporte aucun résultat s'agissant des antibiotiques. Au vu de ce document, il n'est pas évident qu'un antibiotique puisse effectivement migrer hors de la masse. Il est connu que l'éthanol ne migre pas hors de la masse mais reste piégé dans cette dernière probablement du fait de liaison avec les groupements hydroxyle de l'éthylcellulose. De plus, il n'est pas évident au vu de l'enseignement de ce document que l'antibiotique conserve ses propriétés antibiotiques après sa diffusion vers le milieu extérieur. En effet, l'activité antibiotique est souvent liée à la conformation de la molécule et les interactions entre l'antibiotique, l'éthylcellulose et l'éthanol n'étant pas bien connues, il est probable que même si une fraction de la quantité d'antibiotique contenue dans la composition migre vers le milieu extérieur, cette fraction ne soit plus active en tant qu'antibiotique.

La publication de Thawatchai Phaechamud & al, intitulée « Solvent exchange-induced in situ forming gel comprising ethyl cellulose-antimicrobial drugs » et publiée dans la revue International Journal of Pharmaceutics en 2015 (pages 361-392) décrit une composition constituée d'éthylcellulose, de N-méthyl-2-pyrrolidone et d'hyclate de doxycycline (monohydrochlorure hémiéthanolate hémihydrate de doxycycline). La composition forme un gel qui n'est pas stable et qui se délite. Le gel se dégrade rapidement et le solvent migre rapidement hors du gel lequel ne forme plus une masse *in situ.* L'hyclate de doxycycline est également relargué dans le milieu extérieur. Le gel formé temporairement par la composition mise en contact avec une solution saline a des propriétés antimicrobiennes qui proviennent à la fois du relargage de la N-méthyl-2-pyrrolidone et de celui de l'hyclate de doxycycline.

Un but de la présente invention est de proposer une composition pharmaceutique qui soit apte à former une masse solide lorsqu'elle est mise en contact avec une solution saline, la masse étant elle apte à relarguer progressivement l'antibiotique sans altération notable de l'activité antibiotique de ce dernier.

Un autre but de la présente invention est de proposer une composition pharmaceutique qui soit apte à former une masse solide lorsqu'elle est mise en contact avec une solution saline, la masse étant apte à relarguer progressivement l'antibiotique sans s'altérer mécaniquement et/ou chimiquement.

Un autre but est de proposer une composition pharmaceutique qui soit apte à former une masse solide lorsqu'elle est mise en contact avec une solution saline et qui procure un effet sclérosant par contact tout en ayant un effet antibiotique.

La présente invention propose une composition pharmaceutique apte à former une masse solide lorsqu'elle est mise en contact avec une solution saline. De manière caractéristique, selon l'invention, elle contient de l'éthanol, de l'éthylcellulose et au moins un antibiotique non ionique choisi parmi l'amoxicilline, la cefpodoxime la lymécycline, la clarithromycine, le nifuroxazide, le secnidazole et le thiamphenicol.

Ces antibiotiques qui sont des antibiotiques fréquemment utilisés se sont révélés être aptes à :
- être emprisonnés dans la masse solide formée par contact entre la composition contenant de l'éthanol et de l'éthylcellulose et une solution saline ;
- puis être relargués par diffusion hors de la masse vers le milieu extérieur, constitué d'une solution saline.

La masse formée par contact entre le gel d'éthylcellulose et d'éthanol avec une solution saline ne se délite pas et reste solide en dépit du relargage de l'antibiotique.

L'éthanol permet d'avoir un effet sclérosant par contact entre la masse formée *in situ* et les tissus biologique environnants.

La Demanderesse a de plus mis en évidence que l'activité antibiotique des antibiotiques précités n'était pas altérée par l'emprisonnement de l'antibiotique dans la masse solide. En effet, en dépit des nombreuses interactions de type ionique et/ou Van der Waals et/ou liaison hydrogène pouvant avoir lieu dans la masse, notamment avec les hydroxyles de l'éthanol et ceux de l'éthylcellulose, les antibiotiques précités sont, de manière surprenante, relargués sans modification de leur structure ou de leur conformation. Ils demeurent actifs en tant qu'antibiotique et ce même après avoir séjourné un temps certain, de l'ordre de plusieurs semaines ou mois dans la masse ou dans la composition de l'invention.

La Demanderesse a également mis en évidence que l'ajout d'un antibiotique tel que précité (lequel n'est pas ionique, contrairement aux antibiotiques cités dans la publication précitée) ne modifie pas les interactions entre l'éthylcellulose et l'éthanol. En dépit de la présence de l'antibiotique non ionique, on obtient bien une masse solide qui relargue ensuite progressivement l'antibiotique, sans s'altérer mécaniquement ou chimiquement.

Une telle composition est en effet apte à former une masse solide lorsqu'elle est mise au contact avec une solution saline (composant majoritaire d'un tissu physiologique).

Avantageusement, la composition de l'invention est constituée d'eau, d'éthanol, d'éthylcellulose et dudit antibiotique.

Avantageusement, la composition de l'invention présente une viscosité permettant de l'injecter dans un organisme, notamment au moyen d'une seringue (montée d'une aiguille ou d'un cathéter ou encore de former une masse qui reste contenue *in situ* par dépôt direct en cas d'application par chirurgie ouverte). La Demanderesse a constaté que l'ajout d'antibiotique ne modifie pas la viscosité et la rhéologie du mélange éthanol + éthylcellulose. Le placement de l'aiguille ou du cathéter sous contrôle radioscopique permet de placer leur extrémité distale dans le site infectieux. Le mélange constitué du gel à base d'éthanol et d'antibiotique reste injectable ; ce qui permet d'atteindre des foyers infectieux internes, de manière ciblée, par exemple des foyers infectieux situés dans des os. On évite ainsi une prise systémique massive d'antibiotique générale, notamment par voie orale ou intraveineuse.

La composition de l'invention contient au moins 50% en volume d'éthanol et de préférence au moins 90% et au moins 95% en volume d'éthanol.

La composition de l'invention contient une concentration massique dudit antibiotique adaptée à son utilisation. A titre d'exemple, elle peut comprendre une concentration massique dudit antibiotique sensiblement égale ou supérieure à 0,001 g/mL et sensiblement égale ou inférieure à 1g/mL et en particulier sensiblement égale à 0,313g/mL. L'Homme du Métier est à même de procéder à des tests de cinétique de diffusion de l'antibiotique hors de la masse afin de déterminer la quantité d'antibiotique à mélanger avec le mélange éthanol - éthylcellulose en fonction de l'utilisation envisagée.

La présente invention concerne également un produit de combinaison comprenant une composition pharmaceuticalement acceptable comprenant ou constituée d'eau, d'éthanol et d'éthylcellulose et un antibiotique non ionique choisi parmi l'amoxicilline, la cefpodoxime la lymécycline, la clarithromycine, le nifuroxazide, le secnidazole et le thiamphenicol. Ces deux composants du produit de combinaison que sont la composition à base d'éthanol et l'antibiotique peuvent être mélangés pour former, par exemple juste avant l'injection, la composition de l'invention.

Avantageusement, ladite composition pharmaceutique contient au moins 50% en volume d'éthanol et de préférence au moins 90% et au moins 95% en volume d'éthanol.

La présente invention concerne également la masse solide apte à former un implant et obtenue par mise en contact de la composition selon l'invention avec une solution saline.

La présente invention concerne également l'utilisation de la composition pharmaceuticeuticalement acceptable selon l'invention ou du produit de combinaison selon l'invention pour la formation d'un implant dans le corps d'un sujet, ledit implant étant apte à délivrer un antibiotique dans le corps du sujet. Le sujet est de préférence un mammifère et notamment un humain.

La présente invention concerne également la masse solide de l'invention pour son utilisation en tant que médicament pour le traitement des infections chez un sujet, notamment un mammifère en particulier un humain.

La présente invention concerne également la composition selon l'invention pour son utilisation en tant que médicament et notamment en tant que médicament pour le traitement des infections localisées ou des foyers infectieux, en particulier des infections localisées de la peau et du tissu cellulaire sous-cutané ou profonds, des kystes infectieux et plus particulièrement des kystes osseux infectieux. La présente invention concerne également la composition selon l'invention pour son utilisation en tant que médicament pour le traitement des maladies répondant aux antibiotiques, plus particulièrement pour le traitement des sites infectieux, des kystes et des maladies entrainant des modifications pathologiques tissulaires, plus particulièrement les foyers infectieux, les kystes et plus particulièrement les kystes infectieux, les kystes rénaux, les ostéomes ostéoïdes, les tumeurs hépatocellulaires et les angiomes artérioveineux.

L'antibiotique diffusant hors de la masse de l'invention, il va pouvoir agir localement dans le site infectieux ou foyer infectieux où la composition de l'invention a été injectée. L'éthanol va scléroser par contact les tissus environnants.

### DEFINITIONS

Le terme éthanol désigne un mélange d'eau et d'éthanol azéotropique à 20°C et pression atmosphérique, tel que l'éthanol à 96%.

L'Amoxicilline désigne acide 7-[2-amino-2-(4-hydroxyphényl)-acéetyl]amino-3,3-diméthyl-6-oxo-2-thia-5-azabicyclo [3.2.0]heptane-4-carboxylique.

La Cefpodoxime désigne la molécule de formule I suivante :

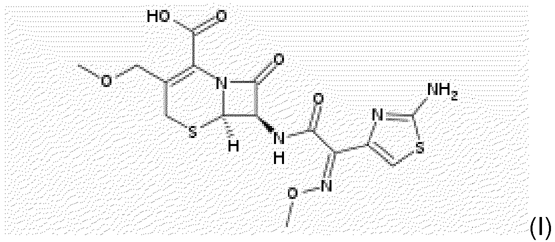

La Lymécycline désigne le composé de formule II suivante :

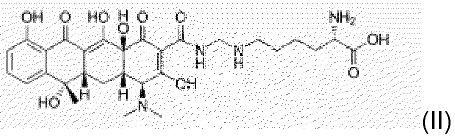

La Clarithromycine désigne le 6-o-méthyl érythromycine A.

Le Nifuroxazide désigne le 4-hydroxy-N-[(5-nitrofuran-2-yl)méthylène]benzohydrazide.

Le Secdinazole désigne le 1-(2-Methyl-5-nitro-1H-imidazol-1-yl)-2-propanol.

Le Thiamphénicol désigne le 2,2-dichloro-N-{(1R,2R)-2-hydroxy-1-(hydroxyméthyl)-2-[4-(méthylsulfonyl)phényl]éthyl}acétamide.

Les antibiotiques utilisés dans le cadre de cette expérimentation ont été présélectionnés pour la sensibilité de la souche *Escherichia Coli* parmi les six familles sur les dix existantes d'antibiotique, notamment pour les Béta lactamines, les Cyclines, les Macrolides, les Nitrofuranes /Nitroimidazoles, les Quinolones, les Phénicolés.

### PARTIE EXPERIMENTALE

### Fabrication de la composition pharmaceutique

La composition pharmaceuticalement acceptable est obtenue en mélange de l'éthylcellulose (Aqualon 100N) à de l'éthanol à 96% (EMPROVE^{®} exp Ph. Eur 1009712500)

La composition F1 présente une concentration massique en éthylcellulose de 0,0678.

La composition F6 présente une concentration massique en éthylcellulose de 0,0584

La composition F12 une concentration massique en éthylcellulose de 0,0500.

Toutes les compositions obtenues forment un gel qui présente une viscosité telle qu'il peut être injecté au moyen d'une seringue.

### Antibiotiques testés

20ml de chaque composition pharmaceutique F1, F6 et F12 a été mélangée à 18°C avec les antibiotiques suivants : Amoxicilline^{®}, Cefpodoxime^{®}, Lymécycline^{®}, Clarithromycine^{®}, Nifuroxazide^{®}, Secnidazole^{®}, Thiamphénicol^{®}. La quantité d'antibiotique utilisée est de 6,26g. Chaque composition selon l'invention contient donc l'antibiotique en une concentration massique de 0,313g/mL.

La température de la salle sous atmosphère contrôlée et à air conditionnée est fixée à 18°C pendant l'expérience.

### Vérification du fait que le mélange composition pharmaceutique + antibiotique reste injectable

20mL de chacune des compositions F1 à F12 ont été mélangés à 6,26g d'amoxicilline en poudre. Pour vérifier la possibilité de prélever et injecter le mélange obtenu on va utiliser une seringue en polycarbonate de 1ml montée d'une aiguille à ailettes de 21G (de diamètre très fin) avec une tubulure de 30 cm. ce qui représente une situation d'extrême difficulté au prélèvement et à l'injection du mélange obtenu. Il a été constaté que même pour la composition F12, il est possible de prélever la composition obtenue contenant l'antibiotique à travers l'aiguille précitée et qu'il est également possible ensuite de l'injecter à 20°C sans résistance particulière du piston et sans obstruction.

### Test de relargage de l'antibiotique et test de conservation de son activité

On utilise un osmoseur comprenant une membrane hémiperméable. D'un côté de la membrane, on place les compositions pharmaceutiques contenant l'antibiotique et de l'autre, on place du sérum physiologique stérile à 9g/L en NaCl.

Pour l'activité antibiotique, on utilise une souche non pathogène d'*E*. *coli* « calibré » Epower^{™} cultivée dans des boîtes de Pétri sur un milieu nutritif TAS agar. Une boîte est conservée en tant que contrôle.

Après mise au contact de la composition pharmaceutique chargée en antibiotique avec le sérum physiologique, on obtient une masse solide. Après 3 heures de contact avec le sérum physiologique dans l'osmoseur, on prélève la solution contenue dans le compartiment de l'osmoseur ne contenant pas la masse solide. On prélève 5ml de cette solution. 0.1mL de cette solution est versée sur une pastille (pour antibiogramme) d'un diamètre de 10 mm², puis et mise en contact avec la souche d'*E*. *coli* précitée dans des boîtes de Pétri. On observe alors l'action de la solution sur les colonies d'*E*. *coli.* On lit l'antibiogramme constitué : on mesure après incubation le diamètre des zones d'inhibition, c'est-à-dire les zones où l'antibiotique a été efficace et a tué la colonie de bactérie. Plus la zone autour de la pastille est grande, plus l'antibiotique a été efficace et donc plus il a tué de bactéries et inversement.

Selon le diamètre de la zone, la bactérie est soit :
- Sensible (si le diamètre est supérieur à 13mm) : l'antibiotique est efficace. Il suffit d'une faible concentration de l'antibiotique en question pour tuer les bactéries.
- Intermédiaire (si le diamètre est compris entre 12mm et 13mm) : l'antibiotique est efficace que dans certaines conditions ;
- Résistante (si diamètre est inférieur à 10mm) : l'antibiotique est inefficace. En effet, la dose nécessaire pour tuer les bactéries est beaucoup trop élevée pour être supportée chez l'homme sans effets secondaires.

L'incubation des boîtes ensemencées avec la bactérie est réalisée à 30°C (+/-1°c) pendant 5 jours. On effectue une lecture du diamètre du disque à partir de 24h. Les expérimentations ont été répétées trois fois.

Le signe + indique l'obtention d'une zone de diamètre supérieur à 13mm dans laquelle la bactérie E. *coli* ne s'est pas développée.

**Tableau 1**

| Composition pharmaceutique | PETRI ((1) | PETRI (2) | PETRI (3) | Moyenne inhibition sur les trois boites mise en culture |
|---|---|---|---|---|
| | Pastille inhibitrice (Oui / Non) | Pastille inhibitrice (Oui / Non) | Pastille inhibitrice (Oui / Non) | |
| F1 + Amoxicilline^{®} A | + | + | + | + |
| F1 + Cefpodoxime^{®} | + | + | + | + |
| F1 + Lymécycline^{®} | + | + | + | + |
| F1 + Clarithromycine^{®} | + | + | + | + |
| F1+ Nifuroxazide^{®} | + | + | + | + |
| F1 + Secnidazole^{®} | + | + | + | + |
| F1+ Thiamphénicol^{®} | + | + | + | + |
| F6 + Amoxicilline^{®} A | + | + | + | + |
| F6 + Cefpodoxime^{®} | + | + | + | + |
| F6 + Lymecycline^{®} | + | + | + | + |
| F6 + Clarithromycine^{®} | + | + | + | + |
| F6+ Nifuroxazide^{®} | + | + | + | + |
| F6 + Secnidazole^{®} | + | + | + | + |
| F6+ Thiamphénicol^{®} | + | + | + | + |
| F12 + Amoxicilline^{®} A | + | + | + | + |
| F12 + Cefpodoxime^{®} | + | + | + | + |
| F12 + Lymecycline^{®} | + | + | + | + |
| F12 + Clarithromycine^{®} | + | + | + | + |
| F12+ Nifuroxazide^{®} | + | + | + | + |
| F12 + Secnidazole^{®} | + | + | + | + |
| F12 + Thiamphénicol^{®} | + | + | + | + |

Dans les boîtes de Pétri témoins de deux types : celle qui contenant de la gélose et une pastille imbibée de sérum physiologique, ou une pastille d'éthanol + éthylcellulose (sans antibiotique), la bactérie s'est développée montrant que l'inhibition de sa croissance est bien due à l'antibiotique (seul) présent dans la solution de sérum physiologique, cet antibiotique provenant lui-même de la masse formée par réaction entre la composition de l'invention et le sérum physiologique.

## Revendications

1. Composition pharmaceutique apte à former une masse solide lorsqu'elle est mise en contact avec une solution saline, ladite composition pharmaceutique contenant au moins 50% en volume d'éthanol, de préférence au moins 90% et au moins 95% en volume d'éthanol, de l'éthylcellulose et au moins un antibiotique, **caractérisé en ce que** ledit antibiotique est un antibiotique non ionique choisi parmi l'amoxicilline, la cefpodoxime la lymécycline, la clarithromycine, le nifuroxazide, le secnidazole et le thiamphenicol.

2. Composition selon la revendication 1, **caractérisée en ce qu'**elle est constituée d'eau, d'éthanol, d'éthylcellulose et dudit antibiotique.

3. Composition selon l'une quelconque des revendications précédentes, caractérisée en que ladite composition présente une viscosité permettant de l'injecter dans un organisme, notamment au moyen d'une seringue.

4. Composition selon l'une quelconque des revendications précédentes, **caractérisée en ce qu'**elle contient une concentration massique dudit antibiotique sensiblement égale ou supérieure à 0,001 g/mL et sensiblement égale ou inférieure à 1g/mL et en particulier sensiblement égale à 0,313g/mL.

5. Composition selon l'une quelconque des revendications 1 à 4, pour son utilisation dans le traitement des infections localisées ou des foyers infectieux.

6. Composition pour son utilisation selon la revendication 5, dans laquelle les infections localisées ou les foyers infectieux sont choisis dans le groupe constitué des infections localisées de la peau et du tissu cellulaire sous-cutané ou profonds, des kystes infectieux et plus particulièrement des kystes osseux infectieux, des sites infectieux et des maladies entrainant des modifications pathologiques tissulaires, plus particulièrement les foyers infectieux, les kystes rénaux, les ostéomes ostéoïdes, les tumeurs hépatocellulaires et les angiomes artérioveineux.

7. Produit de combinaison comprenant une composition pharmaceuticalement acceptable comprenant ou constituée d'eau, au moins 50% en volume d'éthanol et de préférence au moins 90% et au moins 95% en volume d'éthanol, d'éthylcellulose et un antibiotique, **caractérisé en ce que** ledit antibiotique est un antibiotique non ionique choisi parmi l'Amoxicilline, la cefpodoxime, la lymécycline, la clarithromycine, le nifuroxazide le secnidazole et le thiamphenicol.

8. Masse solide apte à former un dispositif de concentration et de relargage d'au moins un antibiotique et obtenue par mise en contact de la composition selon l'une quelconque des revendications 1 à 4 avec une solution saline.

## Patentansprüche

1. Pharmazeutische Zusammensetzung, die geeignet ist, eine feste Masse zu bilden, wenn sie mit einer Kochsalzlösung in Kontakt kommt, wobei die pharmazeutische Zusammensetzung mindestens 50 Vol.-% Ethanol, vorzugsweise mindestens 90 Vol.-% und mindestens 95 Vol.-% Ethanol, Ethylcellulose und mindestens ein Antibiotikum enthält, **dadurch gekennzeichnet, dass** das Antibiotikum ein nichtionisches Antibiotikum ist, ausgewählt aus Amoxicillin, Cefpodoxim, Lymecyclin, Clarithromycin, Nifuroxazid, Secnidazol und Thiamphenicol.

2. Zusammensetzung nach Anspruch 1, **dadurch gekennzeichnet, dass** sie aus Wasser, Ethanol, Ethylcellulose und dem Antibiotikum besteht.

3. Zusammensetzung nach einem der vorhergehenden Ansprüche, **dadurch gekennzeichnet, dass** die Zusammensetzung eine Viskosität aufweist, die es ermöglicht, sie in einen Organismus zu injizieren, insbesondere mittels einer Spritze.

4. Zusammensetzung nach einem der vorhergehenden Ansprüche, **dadurch gekennzeichnet, dass** sie eine Massenkonzentration des Antibiotikums enthält, die im Wesentlichen gleich oder größer als 0,001 g/ml und im Wesentlichen gleich oder kleiner als 1 g/ml und insbesondere im Wesentlichen gleich 0,313 g/ml ist.

5. Zusammensetzung nach einem der Ansprüche 1 bis 4 zur Verwendung bei der Behandlung von lokalisierten Infektionen oder Infektionsherden.

6. Zusammensetzung für die Verwendung nach Anspruch 5, wobei die lokalisierten Infektionen oder Infektionsherde aus der Gruppe ausgewählt sind, bestehend aus lokalisierten Infektionen der Haut und des subkutanen oder tiefen Zellgewebes, infektiösen Zysten und insbesondere infektiösen Knochenzysten, infektiösen Stellen und Erkrankungen, die pathologische Gewebeveränderungen verursachen, insbesondere Infektionsherde, Nierenzysten, osteoide Osteome, Leberzelltumoren und arteriovenösen Angiomen.

7. Kombinationsprodukt, das eine pharmazeutisch akzeptable Zusammensetzung umfasst, die Wasser, mindestens 50 Vol.-% Ethanol, vorzugsweise mindestens 90 Vol.-% und mindestens 95 Vol.-% Ethanol, Ethylcellulose und ein Antibiotikum umfasst oder daraus besteht, **dadurch gekennzeichnet, dass** das Antibiotikum ein nichtionisches Antibiotikum ist, das unter Amoxicillin, Cefpodoxim, Lymecyclin, Clarithromycin, Nifuroxazid, Secnidazol und Thiamphenicol ausgewählt ist.

8. Feste Masse, die geeignet ist, eine Vorrichtung zur Konzentration und Freisetzung von mindestens einem Antibiotikum zu bilden, und durch Inkontaktbringen der Zusammensetzung nach einem der Ansprüche 1 bis 4 mit einer Kochsalzlösung erhalten wird.

## Claims

1. A pharmaceutical composition capable of forming a solid mass when it is brought into contact with a saline solution, said pharmaceutical composition containing at least 50% by volume of ethanol, preferably at least 90% and at least 95% by volume of ethanol, ethylcellulose and at least one antibiotic, **characterised in that** said antibiotic is a nonionic antibiotic selected from amoxicillin, cefpodoxime, lymecycline, clarithromycin, nifuroxazide, secnidazole and thiamphenicol.

2. The composition according to claim 1, **characterised in that** it consists of water, ethanol, ethylcellulose and said antibiotic.

3. The composition according to any one of the preceding claims, **characterised in that** said composition has a viscosity enabling it to be injected into an organism, in particular by means of a syringe.

4. The composition according to any one of the preceding claims, **characterised in that** it contains a mass concentration of said antibiotic substantially equal to or greater than 0.001 g/mL and substantially equal to or less than 1g/mL and in particular substantially equal to 0.313g/mL.

5. The composition according to any one of claims 1 to 4, for use in treating localised infections or infectious outbreaks.

6. The composition for use according to claim 5, wherein the localised infections or infectious outbreaks are selected from the group consisting of localised infections of the skin and subcutaneous or deep cellular tissue, infectious cysts and more particularly infectious bone cysts, infectious sites and diseases leading to pathological changes in tissue, more particularly infectious outbreaks, kidney cysts, osteoid osteomas, hepatocellular tumours and arteriovenous angiomas.

7. A combination product comprising a pharmaceutically acceptable composition comprising or consisting of water, at least 50% by volume of ethanol and preferably at least 90% and at least 95% by volume of ethanol, ethylcellulose and an antibiotic, **characterised in that** said antibiotic is a nonionic antibiotic selected from amoxicillin, cefpodoxime, lymecycline, clarithromycin, nifuroxazide, secnidazole and thiamphenicol.

8. A solid mass capable of forming a device for concentrating and releasing at least one antibiotic and obtained by bringing the composition according to any one of claims 1 to 4 into contact with a saline solution.
